# EUROPEAN PATENT APPLICATION

(11) **EP 1 732 093 A1**
(43) Date of publication of application: **13.12.2006**
(21) Application number: 05720248.3
(22) Date of filing: 08.03.2005
(51) Int. Cl.: H01H 9/20, A61M 5/145, G06F 3/02

(54) **INPUT OPERATION DEVICE**

(30) Priority: 12.03.2004 JP 2004071099
(71) Applicant: Nemoto Kyorindo Co., Ltd., Tokyo 113-0033 (JP)
(72) Inventor: NEMOTO, Shigeru c/o Nemoto Kyorindo Co., Ltd., Bunkyo-ku, Tokyo 1130033 (JP); FUKUDA, Takashi c/o Nemoto Kyorindo Co., Ltd., Bunkyo-ku, Tokyo 1130033 (JP)
(74) Representative: Cabinet Plasseraud
(86) International application number: PCT/JP2005/003976
(87) International publication number: WO 2005/088661

(57) **Abstract**

Manual operation element 401 is covered by element shield member 411 normally openably and closeably supported by member support mechanism 412 and biased to a shield position by shield bias mechanism. In this state, when element shield member 411 is moved to an open position, manual operation element 401 exposed to allow operation. Since element shield member 411 moved to the open position is temporarily held by temporary holding mechanism 424 and then released after the lapse of the time period, manual operation element 401 can be operated by a finger which moves element shield member 401 from the shield position to the open position. Thus, an input operation device can be provided which can be manually operated the manual operation element easily with one hand while it has a configuration that the element shield member automatically covers the manual operation element.

## Description

### Technical Field

The present invention relates to an input operation device that its manual operation element placed on the outer surface of an operation device body is operated by a finger or fingers of an operator, and more particularly, to an input operation device for use in manual operation to start operation of a chemical liquid injector.

### Background Art

Presently available medical apparatuses for capturing diagnostic images of patients include CT (Computed Tomography) scanners, MRI (Magnetic Resonance Imaging) apparatuses, PET (Positron Emission Tomography) apparatuses, SPECT (Single Photon Emission Computed Tomography) apparatuses, ultrasonic diagnostic apparatuses and the like. Angiography apparatuses, MRA (MR angiography) apparatuses and the like are currently used as medical apparatuses for capturing vascular images of patients.

When the abovementioned medical apparatuses are used, a liquid such as a contrast medium or physiological saline may be injected into a patient. Chemical liquid injectors for automatically performing the injection have been put into practical use. A liquid syringe, for example including a cylinder member and a piston member slidably inserted into the cylinder member, is mounted on such a chemical liquid injector. A syringe driving mechanism presses the piston member into the cylinder member.

The cylinder member is filled with a liquid and connected to a blood vessel of a human body near the surface thereof through an extension tube and an injection needle. Thus, the liquid in the liquid syringe is injected with pressure into the blood vessel of the human by the chemical liquid injector. The injection operation of the chemical liquid injector needs to be started in an appropriate timing in association with imaging operation of a diagnostic imaging apparatus. If the injection operation is started in an inappropriate timing, medical malpractice may be caused such as injection of the liquid into a portion of the human body other than the blood vessel.

For this reason, a conventional chemical liquid injector includes a manual operation element such as a push switch which can be turned on to start operation such that the manual operation element is covered with an openable and closable element shield member. An operating error is prevented by allowing operation of the manual operation element only after an operator manually opens the element shield member.

More specifically, the element shield member is slidably supported by a member support mechanism such as a guide rail at a shield position where the manual operation element is shielded and an open position where the element shield member is opened. The element shield member is generally biased to the shield position by a shield bias mechanism such as a coil spring. With this structure, the element shield member usually covers the manual operation element. To operate the manual operation element, an operator needs to move manually the element shield member from the shield position to the open position against the bias of the shield bias mechanism.

Chemical liquid injectors having the input operation device as described above have been manufactured and disclosed on the Internet or the like by the present applicant (see, for example, non-patent documents 1 to 4 below).

Non-patent document 1: "Dual Shot in product guides of Nemoto Kyorindo Co., Ltd" (retrieved in Feb. 13, 2004) (URL:http://www.nemoto-do.co.jp/seihin_ct.html#dual);

Non-patent document 2: "Auto Enhance A-60 in product guides of Nemoto Kyorindo Co., Ltd" (retrieved on Feb. 13, 2004) (URL:http://www.nemoto-do.co.jp/seihin_ct.html#a60);

Non-patent document 3: "Auto Enhance A-25 in product guides of Nemoto Kyorindo Co., Ltd" (retrieved on Feb. 13, 2004) (URL:http://www.nemoto-do.co.jp/seihin_ct.html#a25);

Non-patent document 4: "Sonic Shot 50 in product guides of Nemoto Kyorindo Co., Ltd" (retrieved on Feb. 13, 2004) (URL:http://www.nemoto-do.co.jp/seihin_ang.mr.html.#sonic50)

### Disclosure of the Invention

### Subject to be solved by the Invention

In the input operation devices as described above, after an operator's finger moves the element shield member to the open position from the shield position, the element shield member is immediately returned to the shield position from the open position if the operator's finger is removed from the member. The operator needs to hold the element shield member with the finger until the operation of the manual operation element is finished, which makes it difficult to perform the operation of the input operation device with one hand.

Some input operation devices have an element shield member which is not biased and freely opened or closed. In this case, the operator can open the element shield member and operate the manual operation element with one hand. Such an input operation device, however, requires the operator to close the element shield member manually, and if he forgets to close it, the manual operation element may be operated erroneously.

The present invention has been made in view of the abovementioned problem, and it is an object thereof to provide an input operation device which can be manually operated a manual operation element easily with one hand while it has a configuration that a element shield member automatically covers the manual operation element.

### Means to Solve the Subject

According to an aspect, the present invention provides an input operation device comprising an operation device body, a manual operation element, an element shield member, a member support mechanism, a shield bias mechanism, and a temporary holding mechanism. The manual operation element is placed on the outer surface of the operation device body and operated by a finger of an operator. The element shield member is placed on the outer surface of the operation device body. The member support mechanism openably and closeably supports the element shield member at a shield position where the manual operation element is covered and at an open position where the element shield member is opened to expose the manual operation element. The shield bias mechanism biases the openably and closeably supported element shield member to the shield position. The temporary holding mechanism temporarily holds the element shield member placed at the open position and then releases the element shield member after the lapse of a time period.

Thus, in the input operation device of the present invention, since the element shield member openably and closeably supported by the member support mechanism is usually biased to the shield position by the shield bias mechanism, the element shield member placed at the shield position covers the manual operation element to prevent an operating error of the manual operation element. In this state, when an operator manually moves the element shield member to the open position against the bias of the shield bias mechanism, the manual operation element is exposed to allow operation with a finger of the operator. The element shield member moved to the open position from the shield position is temporarily held by the temporary holding mechanism and then released after the lapse of the time period. If the operator's finger moves the element shield member to the open position from the shield position and removes from the element shield member, the element shield member is not returned to the shield position from the open position immediately.

Various means referred to in the present invention may be arranged to perform their functions, and may comprise dedicated hardware for performing a predetermined function, a data processing apparatus whose predetermined function is given by a computer program, a predetermined function performed in a data processing apparatus according to a computer program, or a combination thereof.

Various components referred to in the present invention do not need to be a separate entity. A plurality of means may be constructed as one member, a certain means may be part of another means, or a certain means may have a portion overlapping a portion of another means.

### Effect of the Invention

In the input operation device of the present invention, the element shield member moved to the open position from the shield position is temporarily held by the temporary holding mechanism and then released after the lapse of the time period. Thus, for example, the operator can move the element shield member to the open position from the shield position with his or her finger, and move his or her finger off the element shield member to operate the manual operation element. In this manner, the input operation device can be operated easily by one hand. In addition, the manual operation element is automatically covered with the element shield member after the lapse of the time period, so that an operating error of the manual operation element can be prevented reliably.

### Brief Description of the Drawings

Fig. 1 is an exploded view showing the structure of a controller unit which is an embodiment of an input operation device according to the present invention;
Fig. 2 is a perspective view showing the outer appearance of the controller unit;
Fig. 3 is a perspective view showing the outer appearance of an internal mechanism of the controller unit;
Fig. 4 is a perspective view showing the outer appearance of a chemical liquid injector;

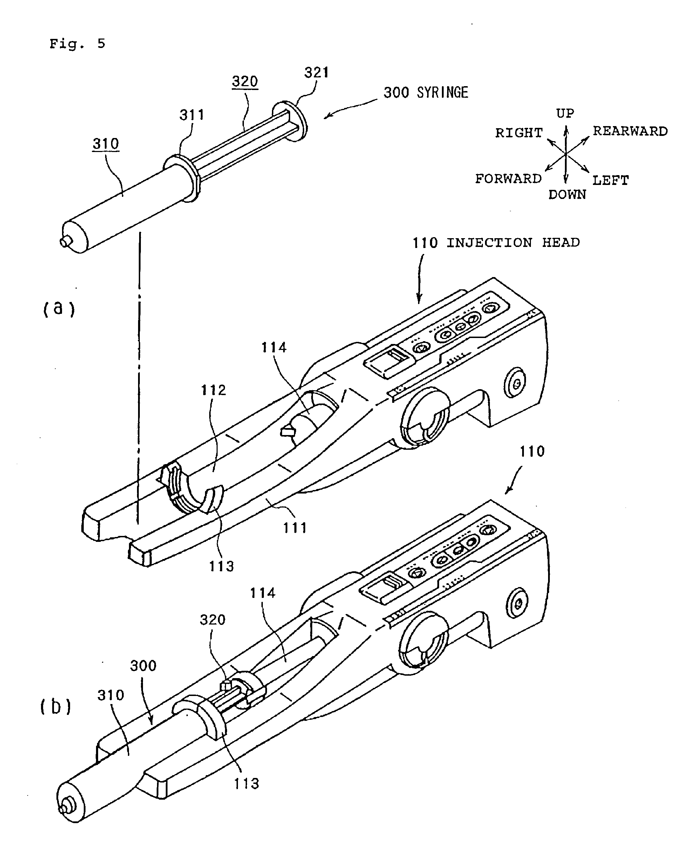

Fig. 5 is a perspective view showing a state for mounting a liquid syringe on an injection head of the chemical liquid injector;
Fig. 6 is a perspective view showing the outer appearance of a diagnostic imaging system; and
Fig. 7 is a block diagram showing the circuit structure of the diagnostic imaging system.

### Description of Reference Numerals

- 100: CHEMICAL LIQUID INJECTOR
- 101: INJECTION CONTROL UNIT CORRESPONDING TO INJECTOR BODY
- 114: SYRINGE DRIVING MECHANISM
- 130: COMPUTER UNIT SERVING AS INJECTION CONTROL MEANS
- 300: LIQUID SYRINGE
- 310: CYLINDER MEMBER
- 320: PISTON MEMBER
- 400: CONTROLLER UNIT SERVING AS INPUT OPERATION DEVICE
- 401: ON-SWITCH SERVING AS MANUAL OPERATION ELEMENT
- 402: OFF-SWITCH SERVING AS SECOND OPERATION ELEMENT
- 410: UNIT HOUSING SERVING AS OPERATION DEVICE BODY
- 411: ELEMENT SHIELD MEMBER
- 412: MEMBER SUPPORT MECHANISM
- 416: COIL SPRING SERVING AS SHIELD BIAS MECHANISM
- 417: NON-SUCKING PORTION
- 418: SUCKING DISC SERVING AS SUCKING MEMBER
- 419: THROUGH-HOLE
- 420: TEMPORARY HOLDING MECHANISM
- 424: DAMPER MECHANISM

### Best Mode for Carrying the Invention

### (Configuration of Embodiment)

An embodiment of the present invention will hereinafter be described with reference to the drawings. While the following description will be made by using the directions of forward, rearward, left, right, up, and down, these directions are defined for convenience in easily explaining the relative relationship between the directions, and the definition does not limit any direction in manufacture or use when the present invention is implemented.

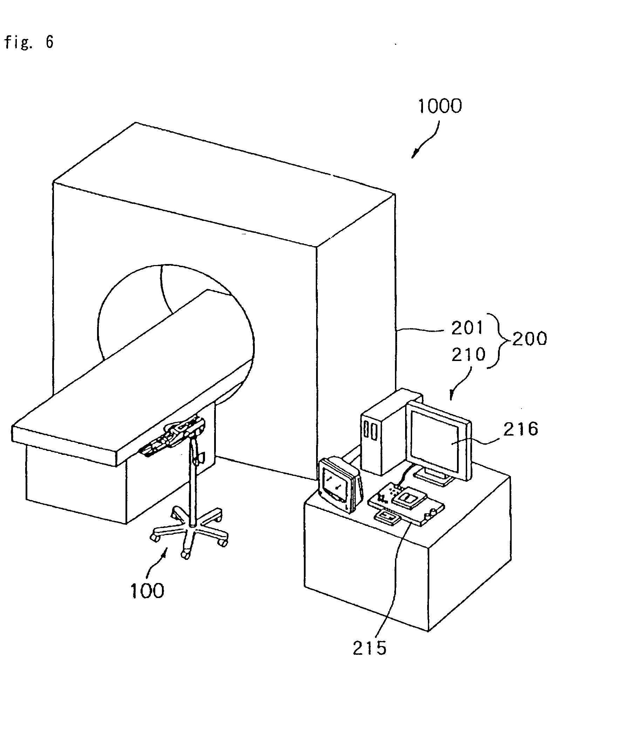

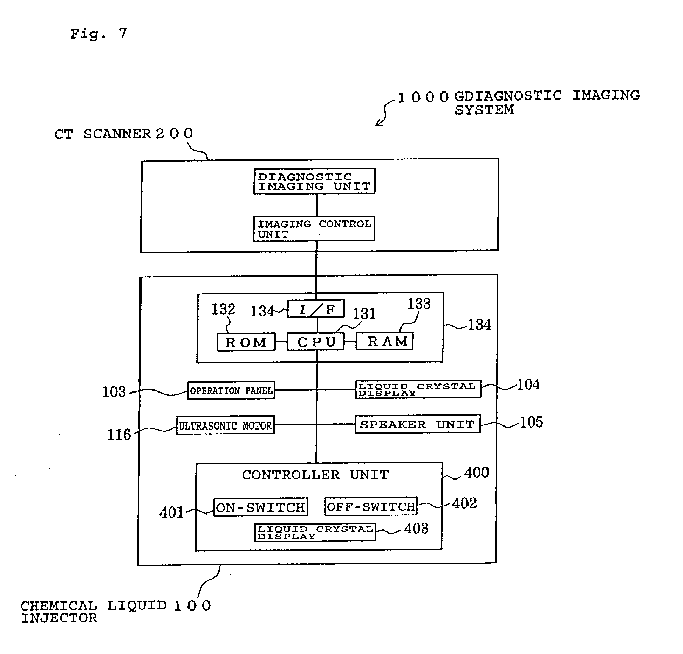

As shown in Figs. 6 and 7, diagnostic imaging system 1000 of the embodiment has chemical liquid injector 100 and CT scanner 200 serving as a diagnostic imaging apparatus. Chemical liquid injector 100 and CT scanner 200 are wire-connected.

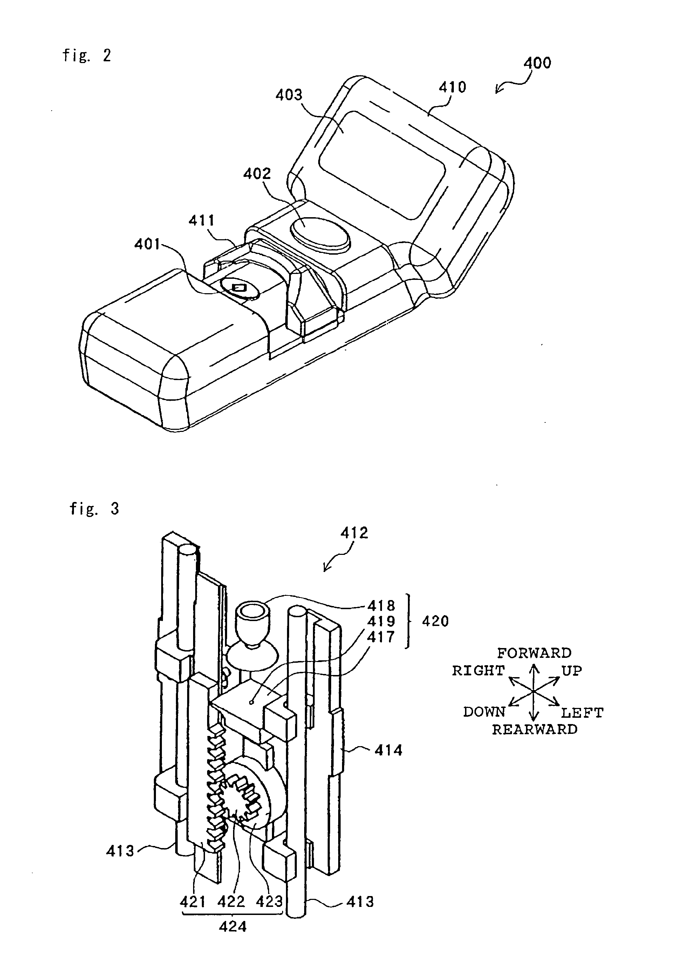

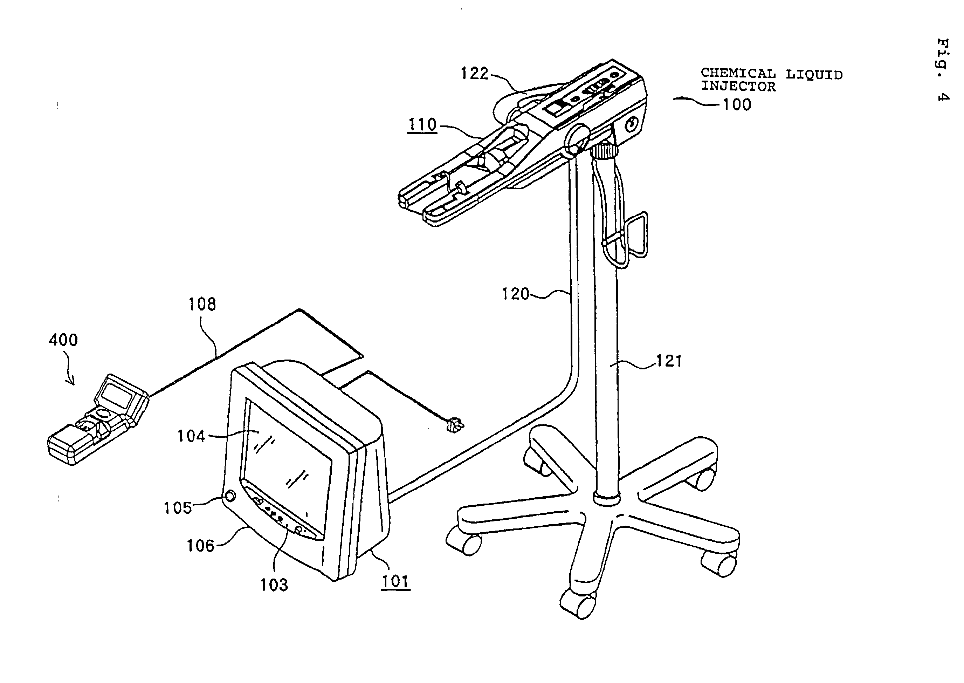

As shown in Fig. 3, chemical liquid injector 100 includes injection control unit 101 corresponding to an injector body and injection head 110 constructed as separate components wherein these are wire-connected through data communication member 120. Injection head 110 is attached to the top end of caster stand 121 by movable arm 122. As shown in Fig. 4, head body 111 of injection head 110 has concave portion 112 as a syringe holding mechanism in the upper surface.

Liquid syringe 300 comprises cylinder member 310 and piston member 320 wherein piston member 320 is slidably inserted into cylinder member 310. Cylinder member 310 and piston member 320 have cylinder flange 311 and piston flange 321 formed in the outer circumferences of the trailing ends thereof, respectively. Cylinder member 310 has conduit 312 formed at the closed leading end surface.

In diagnostic imaging system 1000 of the embodiment, liquid syringe 300 is filled with a contrast medium as a liquid suitable for CT scanner 200. When chemical liquid injector 100 injects the contrast medium into a patient from liquid syringe 300, CT scanner 200 captures diagnostic images of the patient.

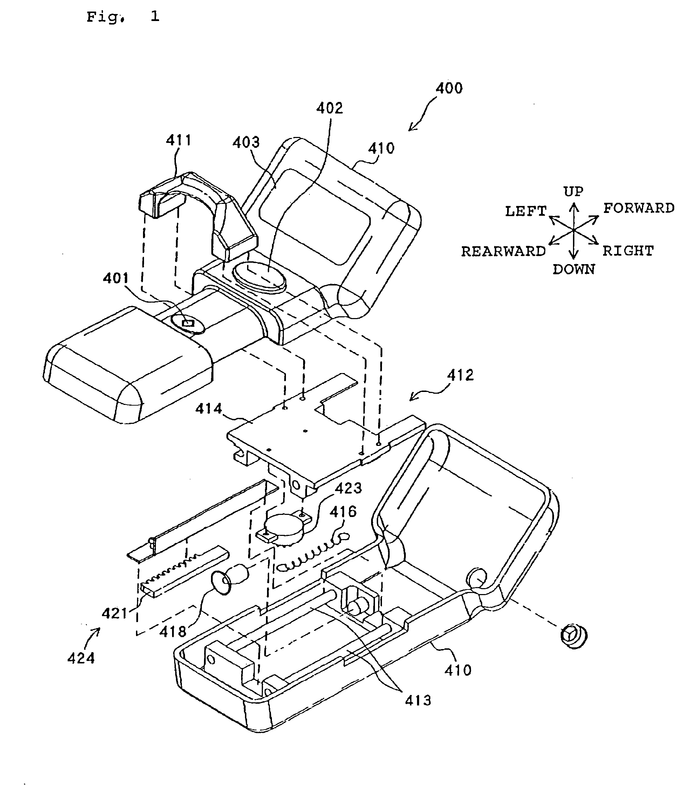

In chemical liquid injector 100 of the embodiment, syringe holding mechanism 113 which is openable or closable is formed in the forward section of concave portion 112 of injection head 110. Syringe holding mechanism 113 removably holds cylinder flange 311 of liquid syringe 300. Syringe driving mechanism 114 is disposed in the rearward section of concave portion 112 of injection head 110. Syringe driving mechanism 114 holds and slides piston member 320 of liquid syringe 300 held in concave portion 112. As shown in Fig. 1, syringe driving mechanism 114 has ultrasonic motor 116 as a driving source and slides piston member 320 with a screw mechanism (not shown) or the like.

On the other hand, as shown in Fig. 3, injection control unit 101 has operation panel 103, liquid crystal display 104, speaker unit 105 and the like, all of which are disposed on the outer face of unit body 106. Controller unit 400, which is formed as a separate component and serves as an input operation device, is wire-connected to injection control unit 101 through connector 108.

Injection control unit 101 contains computer unit 130 serving as an injection control means. Computer unit 130 is connected to respective portions such as operation panel 103, liquid crystal display 104, speaker unit 105, and ultrasonic motor 116.

Computer unit 130 comprises a so-called one-chip microcomputer provided with hardware such as CPU (Central Processing Unit) 131, ROM (Read Only Memory) 132, RAM (Random Access Memory) 133, communication I/F (Interface) 134 and the like. A computer program is installed in ROM 132. CPU 131 performs various types of processing in accordance with the computer program to integrate and control the respective portions of chemical liquid injector 100.

Controller unit 400 has on-switch 401 serving as a manual operation element, an off-switch 402 serving as a second operation element, liquid crystal display 403 and the like. These components are also connected to computer unit 130. When on-switch 401 is operated, computer unit 130 causes syringe driving mechanism 114 to start injection operation. When off-switch 402 is operated, computer unit 130 causes syringe driving mechanism 114 to stop the injection operation.

As shown in Figs. 1 and 2, controller unit 400 has unit housing 410 serving as an operation device body. Unit housing 410 is formed to have an easily portable size in the shape of hollow rectangular parallelepiped which is elongated in the forward-and-rearward direction, flat in the up-and-down direction, and symmetric in the left-and-right direction. The forward portion of unit housing 410 is bent upward. Liquid crystal display 403 is placed on the upper surface thereof.

Off-switch 402 is placed on the upper surface of the forward section of the portion of unit housing 410 that is not bent at the back of liquid crystal display 403. On-switch 401 is disposed on the upper surface at the back of off-switch 402. On/off switches 401, 402 are placed at the center in the left-and-right direction on the upper surface of unit housing 410. Off-switch 402 is larger than on-switch 401.

Element shield member 411 is provided for the upper section of unit housing 410 such that it is slidable in the forward-and-rearward direction by member support mechanism 412. When element shield member 411 is located at the front end of the sliding area corresponding to an open position, on-switch 401 is exposed. When element shield member 411 is located at the back end of the sliding area corresponding to a shield position, on-switch 401 is shielded.

As shown in Figs. 1 and 3, member support mechanism 412 comprises two guide rails 413 and one slider member 414 and is disposed inside unit housing 410. Two guide rails 413 are arranged in parallel in the left-and-right direction such that their longitudinal directions correspond to the forward-and-rearward direction. Slider member 414 is supported slidably in the forward-and-rearward direction by guide rails 413. Slider member 414 is formed in a flat-plate shape which is flat in the up-and-down direction. Both ends of arched element shield member 411 are placed from above on the upper surface of slider member 414 on the left and right.

Coil spring 416 serving as a shield bias mechanism is disposed in parallel with guide rails 413 inside unit housing 410. One end of coil spring 416 is attached to unit housing 410. Since the other end of coil spring 416 is attached to slider member 414, the elastic tension of coil spring 416 biases element shield member 411 to the shield position.

As shown in Fig. 3, slider member 414 has non-sucking portion 417 formed as a convex portion extending downward from the forward portion. Sucking disc 418 serving as an sucking member is located at a forward position opposite to non-sucking portion 417. Sucking disc 418 is attached to unit housing 410 and removably sucks non-sucking portion 417 when element shield member 411 is placed at the open position.

Thus, sucking disk 418 has a concave sucking surface in the rearward section. Non-sucking portion 417 has a smooth surface in the forward section which is sucked by the sucking surface of sucking disc 418. Non-sucking portion 417 has small through-hole 419 for gradually passing outside air into the space between the smooth surface and the sucking surface in close contact with each other. Non-sucking portion 417 and sucking disc 418 construct temporary holding mechanism 420 for temporarily holding element shield member 411 placed at the open position and then releasing it after the lapse of a time period.

Rack member 421 is located in parallel with guide rails 413 inside unit housing 410. Pinion gear 422 which engages with rack member 421 is mounted at the lower surface of slider member 414 via damper unit 423. Since damper unit 423 limits the rotation speed of pinion gear 422 to a predetermined speed, rack member 421, pinion gear 422, and damper unit 423 construct damper mechanism 424 for reducing the speed at which element shield member 411 is moved from the open position to the shield position.

### (Operation of the Embodiment)

The operation of diagnostic imaging system 1000 of the embodiment in the abovementioned structure will be described in order. First, as shown in Fig. 6, injection head 110 of chemical liquid injector 100 is disposed near diagnostic imaging unit 201 of CT scanner 200, and liquid syringe 300 filled with a liquid such as a contrast medium is prepared for use together with an extension tube (not shown) and the like.

Then, liquid syringe 300 is connected to a patient (not shown) in diagnostic imaging unit 201 via the extension tube or the like. Liquid syringe 300 is mounted on injection head 110 of chemical liquid injector 100. In this state, when an operator (not shown) operats on-switch 401 of controller unit 400 with his or her finger, syringe driving mechanism 114 presses piston member 320 into cylinder member 310 of liquid syringe 300 to inject the liquid into the patient from liquid syringe 300.

In chemical liquid injector 100 of the embodiment, element shield member 411 of controller unit 400 is usually biased to the shield position by the elastic force of coil spring 416, so that on-switch 401 is usually covered with element shield member 411 placed at the shield position.

To operate on-switch 401, the operator needs to slide element shield member 411 forward with his or her finger against the elastic force of coil spring 416. Then, sucking disc 418 attached to unit housing 410 sucks non-sucking portion 417 of slider member 414 which is slid together with element shield member 411. That suction holds element shield member 411 at the open position.

Since on-switch 401 near the shield position is exposed in this state, the operator can operate on-switch 401 with his or her finger without being obstructed by element shield member 411. Small through-hole 419 is formed in non-sucking portion 417 sucked by ducking disc 418 as described above, so that the outside air is gradually introduced through the hole 419 into the space between non-sucking potion 417 and ducking disc 418 in close contact with each other.

After the lapse of a sufficient time period necessary for the input operation of on-switch 401, the suction of non-sucking portion 417 by sucking disc 418 is spontaneously released to return element shield member 411 to the shield position from the open position with the elastic force of coil spring 416.

At this point, pinion gear 422 connected to element shield member 411 is rotated and moved along rack member 421 fixed to unit housing 410. Pinion gear 422 is supported on its axis by damper unit 423. Thus, the rotation speed of pinion gear 422 is limited to the predetermined speed to realize the sliding of element shield member 411 from the open position to the shield position at the predetermined low speed.

Off-switch 402 is also mounted on controller unit 400. When the operator operates off-switch 402 with his or her finger, chemical liquid injector 100 forcedly stops the liquid injection. Since liquid crystal display 403 is also mounted on controller unit 400, various types of data, for example the injection state, are output as display thereon.

### (Effect of the Embodiment)

In chemical liquid injector 100 of diagnostic imaging system 1000 of the embodiment, on-switch 401 of controller unit 400 is usually covered with element shield member 411 located at the shield position as described above. This eliminates an operating error such as unnecessary contact of a finger with on-switch 401 and thus prevents start of liquid injection in an inappropriate timing.

When the operator slides element shield member 411 with his or her finger from the shield position to the open position against the elastic force of coil spring 416, element shield member 411 is held at the open position by the suction of non-sucking portion 417 by sucking disc 418. The operator can slide element shield member 411 to the open position and then operate on-switch 401 with the same finger. As a result, the operator can operate controller unit 400 easily with one hand.

Since the suction of non-sucking portion 417 by sucking disc 418 as described above is spontaneously released by the outside air gradually introduced through through-hole 419, element shield member 411 is returned to the shield position from the open position with the elastic force of coil spring 416 after the lapse of the predetermined time period.

On-switch 401 is covered with element shield member 411 only after the lapse of the sufficient time period necessary for the input operation of on-switch 401. Thus, the input operation of on-switch 401 is not hindered. After the lapse of the sufficient time period necessary for the input operation of on-switch 401, on-switch 401 is covered automatically with element shield member 411, which reliably prevents an operating error of on-switch 401.

Temporary holding mechanism 420 for temporarily holding element shield member 411 located at the open position and then releasing it after the lapse of the predetermined time period comprises sucking disc 418 and non-sucking portion 417 having through-hole 419 formed therein without requiring a time circuit or the like, thereby achieving the effects with a simple structure.

Since the speed at which element shield member 411 is returned to the shield position from the open position is limited to the predetermined low speed by damper mechanism 424, element shield member 411 returning automatically does not hit the operator's finger at high speed.

Off-switch 402 is provided alongside of on-switch 401 and is not shielded even when element shield member 411 is slid, so that the operator can make entry to forcedly stop liquid injection at all times. Particularly, since on-switch 401 is small to satisfactorily prevent the start of liquid injection with erroneous operation, while off-switch 402 is large to allow reliable input operation to forcedly stop liquid injection in an emergency.

In controller unit 400 of the embodiment, unit housing 410, element shield member 411, and on/off switches 401, 402 have shapes and arrangements which are symmetrical in the left-and-right direction, and element shield member 411 is slid in the forward-and-rearward direction. Thus, the operator can manually operate controller unit 400 similarly with his or her right hand or left hand.

### (Modifications of the Embodiment)

The present invention is not in any way limited to the abovementioned embodiment, but various changes and modifications may be made therein without departing from the scope of the invention. For example, in the above embodiment, controller unit 400 has the respective portions with the shapes and arrangements symmetric in the left-and-right direction. The respective portions may have shapes and arrangements which are not symmetrical in the left-and-right direction.

In the above embodiment, element shield member 411 is slidably supported at the shield position and the open position by member support mechanism 412. For example, the element shield member may be pivotably supported. In the above embodiment, the sucking member of temporary holding mechanism 420 for removably sucking non-sucking portion 417 comprises sucking disc 418. The sucking member may be formed of an adhesive agent.

In the above embodiment, small through-hole 419 is formed in non-sucking portion 417 to release the suction of non-sucking portion 417 by sucking disc 418 after the lapse of the predetermined time period. It is possible that through-hole 419 is formed in sucking disc 418 or both of non-sucking portion 417 and sucking disc 418. Alternatively, a small concave groove, rather than through-hole 419, may be formed in the smooth surface of non-sucking portion 417 or the sucking surface of sucking disc 418 from the outside to inside. It is also possible that the smooth surface of non-sucking portion 417 and the sucking surface of sucking disc 418 are formed of rough surfaces.

In the above embodiment, non-sucking portion 417 of temporary holding mechanism 420 is formed integrally with element shield member 411, and sucking disc 418 is attached to unit housing 410. Alternatively, it is possible that non-sucking portion 417 is formed integrally with unit housing 410 and sucking disc 418 is attached to element shield member 411, or that a first sucking disc is attached to unit housing 410 and a second sucking disc is attached to element shield member 411.

In the above embodiment, controller unit 400 and injection control unit 101 are formed as separate components which are connected via communication cable 108. For example, it is possible that the controller unit and the injection control unit are integrally formed by mounting the respective portions such as on-switch 401 and element shield member 411 on the injection control unit.

## Claims

1. An input operation device comprising an operation device body and a manual operation element, the manual operation element being placed on an outer surface of the operation device body and operated by a finger of an operator, comprising:
an element shield member placed on the outer surface of the operation device body and shielding the manual operation element;
a member support mechanism openably and closeably supporting the element shield member to a shield position where the manual operation element is covered and to an open position where the element shield member is opened to expose the manual operation element;
a shield bias mechanism biasing the openably and closeably supported element shield member to the shield position; and
a temporary holding mechanism temporarily holding the element shield member placed at the open position and then releasing the element shield member after the lapse of a time period.

2. The input operation device according to claim 1, wherein the temporary holding mechanism comprises a non-sucking portion formed integrally with one of the element shield member and the operation device body, and an sucking member attached to the other of the element shield member and the operation device body and removably sucking the non-sucking portion when the element shield member is placed at the open position.

3. The input operation device according to claim 1, wherein the temporary holding mechanism comprises a first sucking member attached to the element shield member and a second sucking member attached to the operation device body and removably sucking the first sucking member when the element shield member is placed at the open position.

4. The input operation device according to claim 2, wherein the sucking member comprises a sucking disc having a concave sucking surface, the non-sucking member has a smooth surface sucked by the sucking surface of the sucking disc, at least one of the non-sucking portion and the sucking disc has a through-hole formed therein for gradually introducing outside air into space between the smooth surface and the sucking surface in close contact with each other.

5. The input operation device according to claim 2, wherein the sucking member comprises a sucking disc having a concave sucking surface, the non-sucking member has a smooth surface sucked by the sucking surface of the sucking disc, at least one of the non-sucking portion and the sucking disc has a concave groove formed therein for gradually introducing outside air into space between the smooth surface and the sucking surface in close contact with each other.

6. The input operation device according to claim 2, wherein the sucking member comprises a sucking disc having a concave sucking surface, and the non-sucking portion has a rough surface sucked by the sucking surface of the sucking disc.

7. The input operation device according to claim 3, wherein each of the first sucking member and the second sucking member comprises a sucking disc having a concave sucking surface, and at least one of the first sucking member and the second sucking member has a through-hole formed therein for gradually introducing outside air into space between the sucking surfaces in close contact with each other.

8. The input operation device according to claim 3, wherein each of the first sucking member and the second sucking member comprises a sucking disc having a concave sucking surface, and the sucking surface of at least one of the first sucking member and the second sucking member has a concave groove formed therein for gradually introducing outside air into space between the sucking surfaces in close contact with each other.

9. The input operation device according to claim 3, wherein each of the first sucking member and the second sucking member comprises a sucking disc having a concave sucking surface, and the sucking surface of at least one of the first sucking member and the second sucking member is formed of a rough surface.

10. The input operation device according to any one of claims 1 to 9, further comprising a damper mechanism reducing the speed at which the member support mechanism is moved at least from the open position to the shield position.

11. The input operation device according to any one of claims 1 to 10, further comprising a second operation element operated by a finger and provided alongside of the manual operation element at a position where the second operation element is not covered with the element shield member.

12. The input operation device according to any one of claims 1 to 11, wherein the member support mechanism supports the element shield member slidably to the shield position and the open position.

13. The input operation device according to claim 12, wherein the operation device body is formed to be portable in the shape of rectangular parallelepiped symmetrical in a left-and-right direction,
the manual operation element is placed at a position in substantially the center in the left-and-right direction on an upper surface of the operation device body,
the element shield member is formed in a symmetric shape in the left-and-right direction, and
the member support mechanism supports the element shield member slidably in a forward-and-rearward direction and places the element shield member on the upper surface of the operation device body.

14. A chemical liquid injector injecting a liquid into a patient through an extension tube from a liquid syringe including a piston member slidably inserted into a cylinder member, comprising:
a syringe driving mechanism pressing the piston member into the cylinder member;
the input operation device according to any one of claims 1 to 13; and
injection control means for causing the syringe driving mechanism to start operation when the manual operation element of the input operation device is manually operated.

15. The chemical liquid injector according to claim 14, comprising the input operation device according to claim 11,
wherein the injection control means causes the syringe driving mechanism to stop operation when the second operation element is manually operated.

16. The chemical liquid injector according to claim 14 or 15, further comprising an injection device body containing at least the injection control means,
wherein the injector body and the operation device body of the input operation device are formed integrally

17. The chemical liquid injector according to claim 16, further comprising an injector body containing at least the injection control means,
wherein the injector body and the operation device body of the input operation device are formed as separate components,
the injector body and the input operation device are connected via a communication cable, and
the communication cable electrically couples the injection control means with the manual operation element.

18. The chemical liquid injector according to claim 17, comprising the input operation device according to claim 13.
